# NEUE EUROPÄISCHE PATENTSCHRIFT

(11) **EP 1 742 621 B2**
(45) Veröffentlichungstag und Bekanntmachung des Hinweises auf die Entscheidung über den Einspruch: **12.10.2011**
(45) Hinweis auf die Patenterteilung: 27.02.2008
(21) Anmeldenummer: 05735625.5
(22) Anmeldetag: 19.04.2005
(51) Int. Cl.: A61K 9/70

(54) **WIRKSTOFFABGABESYSTEM AUS EINEM WIRKSTOFFHALTIGEN PFLASTER UND MINDESTENS EINEM WIRKSTOFFABGABEREGULIERUNGSMITTEL**
AGENT-RELEASING SYSTEM COMPRISING AN AGENT-CONTAINING PATCH AND AT LEAST ONE MEANS REGULATING THE RELEASE OF AGENTS
SYSTEME DE LIBERATION DE PRINCIPE ACTIF CONSTITUE D'UN TIMBRE ADHESIF CONTENANT LE PRINCIPE ACTIF ET D'AU MOINS UN REGULATEUR DE LIBERATION DE PRINCIPE ACTIF

(30) Priorität: 26.04.2004 DE 102004020463
(43) Veröffentlichungstag der Anmeldung: 17.01.2007
(73) Patentinhaber: Grünenthal GmbH, 52078 Aachen (DE)
(72) Erfinder: KUGELMANN, Heinrich, 52068 Aachen (DE)
(74) Vertreter: Kutzenberger, Helga
(86) Internationale Anmeldenummer: PCT/EP2005/004139
(87) Internationale Veröffentlichungsnummer: WO 2005/102270

(56) Entgegenhaltungen:
- EP-A2- 0 249 475
- WO-A1-88/05293
- DD-A5- 291 700
- DE-A1- 19 733 981
- DE-C2- 19 900 645
- GB-A- 2 356 812
- US-A- 1 602 344
- US-B1- 6 537 571
- US-B1- 6 682 757
- DATABASE WPI Section Ch, Week 199832 Derwent Publications Ltd., London, GB; Class A96, AN 1998-371035 XP002377232 & JP 10 147521 A (YUTOKU YAKUHIN KOGYO KK) 2. Juni 1998 (1998-06-02)
- WOLF-DIETER MÜLLER-JAHNCKE: 'Heidelberger Schriften zur Pharmazie- und Naturwissenschaftsgeschichte', 2001, WISSENSCHAFTLICHE VERLAGSGESELLSCHAFT STUTTGART Artikel ULRIKE ZEBER: 'Die Geschichte des Pflasters', Seiten 249 - 254

## Beschreibung

Die vorliegende Erfindung betrifft ein Wirkstoffabgabesystem zur systemischen oder topischen Abgabe eines Wirkstoffes durch und/oder an die Haut eines menschlichen oder tierischen Organismus umfassend ein wirkstoffhaltiges Pflaster und wenigstens ein teilbares, wirkstoffundurchlässiges oder wenigstens ein teilbares, die Wirkstoffabgabe retardierendes Wirkstoffabgaberegulierungsmittel, das von dem Pflaster separiert vorliegt, oder ein gegebenenfalls teilbares, die Wirkstoffabgabe retardierendes Wirkstoffabgaberegulierungsmittel, das bereits mit dem Pflaster lösbar verbunden ist.

Die topische oder systemische Abgabe eines Wirkstoffes über die Haut an einem menschlichen oder tierischen Körper erfolgt üblicherweise dadurch, dass über die Kontaktfläche zur Haut der Wirkstoff von der Haut aufgenommen wird.

Dabei wird üblicherweise über die gesamte Kontaktfläche des wirkstoffhaltigen Bereichs eines Pflasters der Wirkstoff abgegeben.

Um eine individuelle Dosierung des Wirkstoffes bei einer solchen transdermalen Applikation zu erreichen, wird üblicherweise bei den bekannten wirkstoffhaltigen Matrixpflastern das Pflaster geteilt, z. B. durch Abschneiden. Dies führt meist zu Ungenauigkeiten in der Dosierung. Eine individuelle Dosierung aus Pflastern, die den Wirkstoff in einem Reservoir vorliegen haben, ist auf diese Weise nicht möglich.

Dies gilt auch für die in US 2003/0125659 beschriebene individuelle Dosierungsmöglichkeit von wirkstoffhaltigen Pflastern, die den Wirkstoff in einer Matrix eingebettet enthalten. Ein Nachteil der dort beschriebenen Dosierungssysteme ist, dass die Dosierung nur für Pflaster mit einer bestimmten vorgegebenen Breite, die nämlich der Dosierungsvorrichtung entspricht, geeignet ist. Eine entsprechende Verwendung der auf dem Markt erhältlichen, unterschiedlichen wirkstoffhaltigen Matrixpflaster mit beliebiger Gestaltung ist nach diesem bekannten Dosierungssystem nicht möglich.

Weiterhin ist aus DE 197 33 981 eine individuelle Dosierung eines transdermalen, therapeutischen Systems bekannt, in dem ein Teil des wirkstoffhaltigen Bereichs eines Matrixpflasters mit einer für den Wirkstoff undurchlässigen Deckschicht bedeckt wird. Das System sieht eine einmalige Möglichkeit zur Verminderung der individuellen Dosis der transdermalen Wirkstoffabgabe vor.

US6682757 offenbart mehrere, miteinander verbundene, transdermale therapeutische Systemeinheiten mit einem Hormon als Wirkstoff, die über vorgegebene Trennlinien trennbar sind. Zwischen Wirkstoffschicht und Klebstoffschicht kann eine die Wirkstoffabgabe retardierende Schicht angeordnet sein. Durch die Abtrennung von Systemeinheiten wird eine Verminderung der gesamten Wirkstoffdosis ermöglicht.

Es stellte sich daher die Aufgabe, ein Wirkstoffabgabesystem für wirkstoffhaltige Matrix- oder Reservoir-Pflaster mit beliebiger Gestaltung zur Verfügung zu stellen, das eine individuelle, an den Patienten angepasste z. B. dem Körpergewicht entsprechende Dosierung des Wirkstoffes und eine einfache Handhabung erlaubt, wobei mit Hilfe eines einzigen Wirkstoffabgaberegulierungsmittels eine unterschiedlich große Verminderung der Dosierung eingestellt und/oder eine Verabreichung des Wirkstoffes mit einer individuellen, an die Bedürfnisse des Patienten angepasste Freisetzung des Wirkstoffes erzielt werden kann.

Diese Aufgabe wird durch Bereitstellung des erfindungsgemäßen Wirkstoffabgabesystems zur systemischen oder topischen Abgabe eines Wirkstoffes durch und/oder an die Haut eines menschlichen oder tierischen Organismus erreicht.,
das
a) ein wirkstoffhaltiges Pflaster und
b) wenigstens ein teilbares, wirkstoffundurchlässiges oder wenigstens ein teilbares, die Wirkstoffabgabe retardierendes Wirkstoffabgaberegulierungsmittel, das von dem Pflaster separiert vorliegt, oder ein gegebenenfalls teilbares, die Wirkstoffabgabe retardierendes Wirkstoffabgaberegulierungsmittel, das bereits mit dem Pflaster lösbar verbunden ist,
umfasst.

Vorzugsweise weist das teilbare, wirkstoffundurchlässige, vom Pflaster separiert vorliegende Wirkstoffabgaberegulierungsmittel folgenden Schichtaufbau auf:
- eine lösbare Schutzschicht (1)
- eine Klebeschicht (1)
- eine Wirkstoffsperrschicht
- gegebenenfalls eine weitere Klebeschicht (2) und
- gegebenenfalls eine weitere lösbare Schutzschicht.(2),
wobei
die gegebenenfalls vorhandene weitere Klebeschicht (2) zum Verbinden mit der Haut bei der Applikation dient.

Dabei wird nach Ablösen der lösbaren Schutzschicht (1) mit Hilfe der Klebeschicht (1) das gewünschte individuell angepasste Ausmaß des wirkstoffhaltigen Bereichs des Pflasters mit Hilfe des erfindungsgemäßen Wirkstoffabgaberegulierungsmittels enthaltend eine Wirkstoff-Sperrschicht abgedeckt. Vorteilhafterweise weist dieses wirkstoffundurchlässige Wirkstoffabgaberegulierungsmittel auch noch eine weitere Klebeschicht (2) zum Verbinden mit der Haut nach Ablösen der weiteren lösbaren Schutzschicht (2) auf, so dass die Wirkstoffsperrschicht zwischen zwei Klebeschichten vorliegt, wodurch eine Kontaktunterbrechung des Pflasters zur Haut vermieden wird.

Vorteilhafterweise weist ein solches Wirkstoffabgaberegulierungsmittel auch zur Erleichterung der Handhabung bei der Abtrennung eines Teils des Wirkstoffabgaberegulierungsmittels zur individuellen Dosierung des Wirkstoffabgabe, d. h. zur Einstellung einer unterschiedlich verminderten Dosierung des Wirkstoffes, die weitere Klebeschicht (2) und die damit verbundene weitere lösbare Schutzschicht (2) auf. Diese Handhabung wird zusätzlich durch die vom Pflaster separierte Verpackung des Wirkstoffregulierungsmittels erleichtert.

Wie bereits erwähnt, kann mit Hilfe des separat verpackten, teilbaren wirkstoffundurchlässigen Wirkstoffabgaberegulierungsmittels durch individuelle Teilung des Mittels die Wirkstoffabgabe in einem beliebigen Ausmaß, vorzugsweise durch entsprechendes Abdecken des wirkstoffhaltigen Bereichs des Pflasters die Wirkstoffabgabe gesperrt und damit individuell dosiert werden.

Alternativ kann das erfindungsgemäße Wirkstoffabgabesystem gemäß Anspruch 1 kein wirkstoffundurchlässiges Wirkstoffabgaberegulierungsmittel, sondern ein gegebenenfalls teilbares, die Wirkstoffabgabe retardierendes Wirkstoffabgaberegulierungsmittel umfassen.

Vorzugsweise ist ein solches, die Wirkstoffabgabe retardierendes, gegebenenfalls teilbares Wirkstoffabgaberegulierungsmittel mehrschichtig und umfasst folgenden Schichtaufbau:
- eine lösbare Schutzschicht (1)
- eine gegebenenfalls die Wirkstoffabgabe retardierende Klebeschicht (1)
- gegebenenfalls eine die Wirkstoffabgabe gegebenenfalls weiter retardierende Schicht (3)
- gegebenenfalls eine weitere Klebeschicht (2') und
- gegebenenfalls eine weitere lösbare Schutzschicht (2').

Vorzugsweise ist der Schichtaufbau eines erfindungsgemäßen, gegebenenfalls teilbaren, die Wirkstoffabgabe retardierenden Wirkstoffabgaberegulierungsmittels mehrschichtig mit folgendem Schichtaufbau:
- eine lösbare Schutzschicht (1)
- eine Klebeschicht (1)
- eine die Wirkstoffabgabe retardierende Schicht (3)
- eine weitere Klebeschicht (2') und
- eine weitere lösbare Schutzschicht (2').

Das erfindungsgemäße, die Wirkstoffabgabe retardierende, teilbare Wirkstoffabgaberegulierungsmittel weist über der Klebeschicht (1) eine lösbare Schutzschicht (1) auf, wenn es von dem Pflaster separiert vorliegt. Dies erleichtert die Handhabung des Mittels, inbesondere bei seiner Teilung. Die weitere Klebeschicht (2') und lösbare Schutzschicht (2') liegen vor, um das Pflaster nach Entfernen der Schutzschicht (2') bei der Applikation mit der Haut eines menschlichen oder tierischen Organismus zu verbinden.

Dementsprechend weist das erfindungsgemäße, teilbare, die Wirkstoffabgabe retardierende Wirkstoffabgaberegulierungsmittel folgenden Schichtaufbau auf, wenn es vom Pflaster separiert vorliegt:
- eine lösbare Schutzschicht (1)
- eine Klebeschicht (1)
- eine die Wirkstoffabgabe retardierende Schicht (3)
- eine weitere Klebeschicht (2') und
- eine weitere lösbare Schutzschicht (2').

Sofern die beiden Klebeschichten (1) und (2') dick genug sind, können diese unter Weglassung der die Wirkstoffabgabe retardierenden Schicht (3) als eine entsprechende die Wirkstoffabgabe retardierende Schicht funktionieren.

Dies gilt selbstverständlich auch für den Aufbau eines gegebenenfalls teilbaren, die Wirkstoffabgabe retardierenden Wirkstoffabgaberegulierungsmittels, das mit dem Pflaster bereits lösbar verbunden ist.

Vorzugsweise weist ein solches Wirkstoffabgaberegulierungsmittel folgenden Schichtaufbau auf:
- eine lösbare Schutzschicht (1), die zumindest mit einem Teilbereich des Pflasters, vorzugsweise zumindest mit dem wirkstoffhaltigen Bereich des Pflasters
und
- auf ihrer anderen Oberfläche mit einer Klebeschicht (1) jeweils lösbar verbunden ist,
- eine an die Klebeschicht (1) angrenzende, die Wirkstoffabgabe retardierende Schicht (3)
- eine weitere Klebeschicht (2')
- eine weitere lösbare Schutzschicht (2')

Vorzugsweise weist das mit dem Pflaster bereits lösbar verbundene, gegebenenfalls teilbare Wirkstoffregulierungsmittel zur besseren Handhabung die weitere lösbare Schutzschicht (2') auf, die insbesondere die Handhabung des Mittels während der Teilung erleichtert.

Zur Applikation des erfindungsgemäßen Wirkstoffabgabesystems wird von dem Mittel, nach seiner Teilung, sofern es separiert von dem Pflaster vorliegt, die lösbare Schutzschicht (1) zunächst entfernt und die Klebeschicht (1) zumindest mit einem Teilbereich des wirkstoffhaltigen Bereichs des Pflasters verbunden oder das mit dem Pflaster lösbar verbundene Mittel vom Pflaster insgesamt abgelöst, gegebenenfalls geteilt, die Schutzschicht (1) abgezogen und die Klebeschicht (1) mit zumindest einem Teilbereich des wirkstoffhaltigen Bereichs des Pflasters verbunden. Vor der Applikation wird dann die weitere lösbare Schutzschicht (2') von der weiteren Klebeschicht (2') entfernt, so dass auch dieser retardierte Bereich des Pflasters in unmittelbaren Hautkontakt gebracht werden kann.

Sowohl das wirkstoffhaltige Pflaster als auch das Wirkstoffabgaberegulierungsmittel können jede beliebige Form, Größe und Farbgebung aufweisen. Beispielsweise können sie unabhängig voneinander eine runde, ovale, rechteckige oder eine andere unregelmäßige Form aufweisen. Vorzugsweise weist das Pflaster und das Wirkstoffabgaberegulierungsmittel des erfindungsgemäßen Wirkstoffabgabesystems zueinander passende Formen auf.

Die Größe eines erfindungsgemäßen Wirkstoffabgaberegutierungsmittels kann vorzugsweise bis zu 100% der Fläche des wirkstoffhaltigen Bereichs des Pflasters betragen.

Sofern das vom Pflaster separiert vorliegende Wirkstoffabgaberegulierungsmittel eine Wirkstoffsperrschicht aufweist, kann die Größe des Mittels dem gesamten wirkstoffhaltigen Bereich des Pflasters entsprechen, da das Mittel durch Teilung auf die gewünschte, der individuellen Dosierung entsprechende Größe gebracht wird. Vorzugsweise werden dann 90% bis 10% des wirkstoffhaltigen Bereichs des Pflasters durch das Wirkstoffabgaberegulierungsmittel abgedeckt, wodurch eine Reduzierung des Wirkstoffabgabe auf 10 bis 90% erreicht werden kann. Die Teilbarkeit des Wirkstoffabgaberegulierungsmittels, das mit einer Wirkstoffsperrschicht ausgestattet ist, gestattet es auf einfache Art und Weise, die individuell gewünschte Dosierung einzustellen, wobei dies durch ein vom Pflaster separiertes Wirkstoffabgaberegulierungsmittels noch erleichtert wird.

Die Größe eines die Wirkstoffabgabe retardierenden Wirkstoffabgaberegulierungsmittels kann bis zu 100% des gesamten wirkstoffhaltigen Bereichs eines Pflasters ausmachen. In einer bevorzugten Ausführung ist die mögliche Bedeckung 100%. In einer weiteren Ausführung ist das lösbar verbundene Wirkstoffabgaberegulierungsmittel auch teilbar, um gegebenenfalls auch eine nicht mit dem Wirkstoffabgaberegulierungsmittel retardierte Freisetzung des Wirkstoffes zumindest in Teilbereichen des wirkstoffhaltigen Bereichs des Pflasters zu erzielen. Zur besseren Handhabung ist das teilbare, die Wirkstoffabgabe retardierende Mittel separiert vom Pflaster, vorzugsweise separiert verpackt.

Sofern das Wirkstoffabgaberegulierungsmittel mit einer retardierenden Schicht ausgerüstet ist, liegt nicht nur ein teilbares Wirkstoffabgaberegulierungsmittel vor, sondern mehrere voneinander separierte entsprechende Wirkstoffabgaberegulierungsmittel, die eine unterschiedliche Größe aufweisen. Diese unterschiedlichen Größen entsprechen dann jeweils dem gewünschten abzudeckenden und damit zu retardierenden Anteil des wirkstoffhaltigen Bereichs des Pflasters. Die unterschiedlichen Größen der voneinander und von dem Pflaster separiert verpackten Wirkstoffabgaberegulierungsmittel sind dann vorzugsweise so gestaltet, dass 10 bis 90% des wirkstoffhaltigen Bereichs des Pflasters abgedeckt werden, damit dort eine retardierte Abgabe des Wirkstoffes erreicht wird.

Um die Abtrennung der Teilbereiche eines teilbaren Wirkstoffabgaberegulierungsmittels zu erleichtern, weist das Wirkstoffabgaberegulierungsmittel vorgegebene Trennmöglichkeiten auf. Diese Trennmöglichkeiten sind vorzugsweise als Schwächungslinien, Perforationen oder Schneidemarkierungen ausgeführt.

Vorzugsweise weisen die voneinander trennbaren Teile eines teilbaren Wirkstoffabgaberegulierungsmittels oder die voneinander separiert vorliegenden Wirkstoffabgaberegulierungsmittel eine unterschiedliche Markierung zur Unterscheidung auf. Diese Markierung zur Unterscheidung kann eine unterschiedliche Färbung und/oder Codierung der voneinander abtrennbaren Teile oder voneinander separat verpackten Mittel sein.

Vorzugsweise wird das erfindungsgemäße Wirkstoffabgabesystem als Kit umfassend das wirkstoffhaltige Pflaster und das Wirkstoffabgaberegulierungsmittel bzw. die Wirkstoffabgabcregulierungsmittel zur Verfügung gestellt. Sofern das Wirkstoffabgaberegulierungsmittel nicht bereits lösbar mit dem Pflaster verbunden ist, ist es vom Pflaster separiert verpackt ein Bestandteil des erfindungsgemäßen Kits.

Das erfindungsgemäße Wirkstoffabgabesystem eignet sich für die Applikation jedes systemisch oder topisch applizierbaren Wirkstoffes. Vorzugsweise eignet sich das erfindungsgemäße Wirkstoffabgabesystem zur transdermalen und/oder topischen Verabreichung von pharmazeutischen Wirkstoffen jeder Art. Insbesondere eignet sich das Pflaster zur transdermalen Abgabe von wenigstens einem pharmazeutischen Wirkstoff aus der Gruppe umfassend Analgetica, Lokalanästhetica, Hormone, Kontrazeptiva, Impfstoffe, Immunmodulatoren, Antiallergica, Antihistaminica, Cardiaca, Antihypertonica, Psychopharmaca, Antirheumatica und Enzyme, bevorzugt zur Verabreichung wenigstens eines pharmazeutischen Wirkstoffes ausgewählt aus der Gruppe umfassend Narkotika, Opioide, Tranquillantien, vorzugsweise Benzodiazepine, Stimulantien und andere Betäubungsmittel.

Besonders bevorzugt eignet sich das erfindungsgemäße Abgabesystem zur Verabreichung mindestens eines transdermal verabreichbaren Opioids, Tranquillanz oder eines anderen Betäubungsmittels, das ausgewählt ist aus der Gruppe umfassend N-{1-[2-(4-Ethyl-5-oxo-2-tetrazolin-1-yl)ethyl]-4-methoxymethyl-4-plperidyl}propionanilid (Atfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-a-Methylphenethylamln (Amfetamin), 2-(a-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6H-thieno[3,2-f][1,2,4]triazolo[4,3-a][1,4]diazepin (Brotizolam), 17-Cyclopropylmethyl-4,5a-epoxy-7a[(S)-1-hydroxy-1,2,2-trimethyl-propyl]-6-methoxy-6,14-endo-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1S,2S)-2-Amino-1-phenyl-1-propanol (Cathin / D-Norpseudoephedrin). 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazapam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3H)-on(Clotiazepam), 10-Chlor 11b-(2chlorphenyl)-2,3,7,11b-tetrahydrooxazolo[3,2-d][1,4]benzodiazepin-6(5H)-on (Cloxazolam), (-)-Methyl-[3ß-benzoyloxy-2ß(1aH,5a*H*)-tropancarboxylat] (Cocain), 4,5a-Epoxy-3-msthoxy-17-methyl-7-morphinen-6a-ol (Codein), 5-(1-Cyclohexenyl)-5-ethylbarbitursäure (Cyclobarbkal), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dextromethorphan, Dezocin. Diampromid, Diamorphon. 7-Chlor-1-methyl-5-phonyl-1H-1,4-benzodiazepin-2(3H)-on (Diazepam), 4,5a-Epoxy-3-methoxy-17-methyl-6a-morphinanol (Dihydrocodein), 4,5α-Epoxy-17-methyl-3,6a-morphinandiol (Dihydramorphin). Dimenoxadol, Dimepheptanol, Dimethylthiambuten, Dioxaphetylbutyrat, Dipipanon, (6a*R*,10a*R*)-6,6,9-Trimethyl-3-pentyl-6a,7,8,10a-tetrahydro-*6H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1H-1.4 benzodiazepin-3-carboxylat] (Ethylloflazepat), 4,5α-Epoxy-3-ethoxy-17-methyl-7-morphinen-6α-ol (E-thylmorphin), Etonitazen, 4,5a-Epoxy-7α-(1-hydroxy-1-methylbutyl)-6-methoxy-17-methyl-6,14-endo-etheno-morphinan-3-ol (Etorphin). *N*-Ethyl-3-phenyl-8,9,10-trinorboman-2-ylamin (Fencamfarnin), 7-[2-(α-Methylphenethylamino)ethyl]-theophyllin) (Fenethyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H-*1,4-benzodiazepin-2(3*H*)-on (Fludiazepam), 5-(2-Fluorphenyl)-1-methyl-7-nitro-1*H-*1,4-benzodiazepin-2(3*H*)-on (Flunitrazepam), 7-Chlor-1-(2-diethymlaminoethyl)-5-(2-fluorphenyl)-1*H*-1,4-benzodiazepin-2(3H)-on (Flurazepam), 7-Chlor-5-phenyl-1-(2,2.2-trifluorethyl)-1*H*-1,4-benzodiazepin-2(3*H*)-on (Halazepam), 10-Brom-11b-(2-fluorphenyl)-2,3,7,11b-tatrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5a-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3S,6S)-6-Dimethylamino-4,4-diphenylheptan-3-ylacetat (Levoacetylmethadol (I.AAM)), (-)-6-Dimethylamino-4,4-diphenyl-3-heptanon (Levomethadon), (-)-17-Methyl-3-morphinanol (Levorphanol), Levophenacylmorphan, Levoxemacin, Lofentanil, 6-(2-Chlorphenyl)-2-(4-methyl-1-piperazinylmethylen)-8-nitro-2H-midazo[1,2-a][1,4] benzodiazepin-1(4H)-on (Loprazolam), 7-Chlor-5-(2-chlorphenyl)-3-hydroxy-1H-1,4-benzodiazepin-2(3*H*)-on (Lorazepam), 7-Chlor-5-(2-chlorphenyl)-3-hydroxy-1-methyl-1*H*-1,4-benzodiazepin-2(3H)-on (Lormetazepam), 5-(4-Chlorphenyl)-2,5-dihydro-3*H*-imidazo[2,1-a]Isoindol-5-ol (Mazindol), 7-Chlor 2,3-dihydro-1-methyl-5-phenyl-1H-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-o-tolyl-4-(3H)-chinazolinon (Methaqualon), Methyl-[2-phenyl-2-(2-piperidyl)acetat] (Methylphenidat), 5-Ethyl-1-methyl-5-phenylbarbüursäure (Methylphenobarbital), 3,3-Diethyl-5-methyl-2,4-piperidindion (Methyprylon), Metopon, 8-Chlor-6-(2-fluorphenyl)-1-methyl-4H-imidazo[1,5-a][1,4]benzodiazepin (Midazolam), 2-(Benzhydrysulfinyl)acetamid (Modafinil), 4,5α-Epoxy-17-methyl-7-morphinen-3,6α-diol (Morphin), Myrophin, (±)-trans-3-1,1-Dimethytheptyl)-7,8,10,10α-tetrahydro-1-hydroxy-6,6-dimethyl-6H-dibenzo[*b*, *d*]pyran-9(6αH)-on (Nabilon), Nalbuphen, Nalorphin, Narcein, Nicomorphin, 1-Methyl-7-nitro-5-phenyl-1H-1,4-benzodiazepin-2(39)-on (Nimetazepam), 7-Nitro-5-phenyl-1*H*-1,4-benzodiazepin-2(3*H*)-on (Nitrazepam), 7-Chlor-5-phenyl-1*H-1,4-*benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3H)-on (Oxazepam), *(cis-trans)-*10-Chlor-2,3,7,11 b-tetrahydro-2-methyl-11b-phenyloxazolo[3,2-d][1,4] benzodiazepin-6-(*5H*)-on (Oxazolam), 4,5α-Epoxy-14-hydroxy-3-methoxy-17-methyl-6-morphinanon (Oxycodon), O-xymorphon, Pflanzen und Pflanzenteile der zur Art Papaver somniferum (einschließlich der Unterart setigerum) gehörenden Pflanzen, Papaveretum, 2-Imino-5-phenyl-4-oxazolidinon (Pemolin), 1,2,3,4.5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-rnethyl-4-phenyt-4-piperidincarboxylat) (Pethidin), Phenadoxon, Phenomorphan, Phenazocin, Phenoperidin, Piminodin, Pholcod in, 3-Methyl-2-phenylmorpholin (Phenmetrazin), 5-Ethyl-5-phenylbarbitursäure (Phenobarbital), α,α-Dimethylphenethylarnin (Phentermin), 7-Chlor-5-phenyl-1-(2-propinyl)-1*H-1*,4-benzodiazepin-2(3H)-on (Pinazepam), α-(2-Pipendyl)benzhydrylalkohol (Pipradrol), 1'-(3-Cyan-3,3-diphenylpropyl)[1,4'-bipipendin]-4'-carboxamid (Piritramid), 7-Chlor-1 - (cyclopropylmethyl)-5-phenyl-1H-1,4-benzodiazepin-2(3H)-on (Prazepam), Premethad on, Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-sec-Butyl-5-ethytbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1H-1,4-benzodiazepin-2(3H)-on (Temazepam), 7-Chlor-5(1-cyclohexenyl)-1-methyl-1*H*-1,4-benzodiazepin-2-(3H)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1R*,2R*)-3-(3-Dimethylamino-1-ethyl-2-methyl-propyl)-phenol, (1R, 2R, 4S)-2- [Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1RS, 3RS, 6RS)-6-Dimethylaminomethyl-1-(3-methoxy-phenyl)-cyclohexan-1,3-diol, 3-(2-Dimethylaminomethyl-1 -hydroxy-cyclohexyl)-phenyl2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1 - enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxycyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie deren entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt deren Hydrochloride.

Die Verbindungen (1 R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol oder deren stereoisomere Verbindungen oder deren physiologisch verträgliche Verbindungen, insbesondere deren Hydrochloride, deren Derivate, wie Ester, Ether oder Amide sowie Verfahren zu ihrer Herstellung sind beispielsweise aus EP-A-693 475 bzw. EP-A-780 369 bekannt. Die entsprechenden Beschreibungen werden hiermit als Referenz eingeführt und gelten als Teil der Offenbarung.

Als transdermal zu verabreichende Wirkstoffe werden vorzugsweise Opioide eingesetzt. Ganz besonders bevorzugt sind diese Wirkstoffe, ausgewählt aus der Gruppe umfassend Morphin, Oxycodon, Buprenorphin und Fentanyl, deren Derivate, vorzugsweise Ester, Ether oder Amide oder deren jeweils physiologisch verträglichen Verbindungen, vorzugsweise deren Salze oder Solvate, besonders bevorzugt deren Hydrochloride.

Bevorzugt liegt die Konzentration des Wirkstoffes bei seiner Sättigungskonzentration oder geringfügig darunter, da dadurch die Abgabe an die Haut gefördert wird. Diese Sättigungskonzentration kann durch Routineversuche ermittelt werden.

Die Wirkstoffsperrschicht des erfindungsgemäßen Wirkstoffabgaberegulierungsmittels besteht vorzugsweise aus einem Polymeren wie Polyester, z. B. Polyethylenterephthalat, Polyolefin, wie Polyethylen, Polypropylen oder Polybutylen, Polycarbonat, Polyethylenoxid, Polyurethan, Polystyrol, Polyamid, Polyimid, Polyvinylacetat, Polyvinylchlorid, Polyvinylidenchlorid, und/oder Copolymere aus z. B. Acrylonitril/Butadien/Styrol. Die Dicke der Schicht beträgt vorzugsweise 5 bis 25 µm.

Die die Wirkstoffabgabe retardierende Schicht eines erfindungsgemäßen Wirkstoffabgaberegulierungsmittels kann aus einem filmbildenden Polymeren ausgewählt aus der Gruppe umfassend Cellulose-Derivate, wie Ethylcellulose, Hydroxypropylcellulose oder Carboxymethylcellulose, Polyethylene, chlorierte Polyethylene, Polypropylene, Polyurethane, Polycarbonate, Polyacrylsäureester, Polyacrylate, Polymethacrylate, Polyvinylalkohole, Polyvinylchloride, Polyvinylidenchloride, Polyvinylpyrrolidone, Polyethylenterephthalate, Polytetrafluoroethylene, Ethylen/Propylen Copolymere, Ethylen/Ethylacrylat Copolymere, Ethylen/Vinylacetat Copolymere, Ethylen/Vinylalkohol Copolymere, Vinylchlorid/Vinylacetat Copolymere, Vinylpyrrolidon/EthylenNinylacetat Copolymere, Kautschuke, gummiartige, synthetische Homo-, Co- oder Blockpolymere, Silikone, Silikon-Derivate und deren Mischungen bestehen.

Als eine die Wirkstoffabgabe retardierende Schicht wird vorzugsweise eine Schicht basierend auf einem Ethylen/Vinylacetat Copolymerisat, einem Polyacrylat oder eine aus zwei Klebeschichten kombinierte Schicht unter Wegfall einer separaten Wirkstoffabgabe retardierenden Schicht eingesetzt, die für die vorgegebene Zeit des Pflastergebrauchs, vorzugsweise bei 3 bis 7 Tagen die Wirkstoffabgabe retardiert. Übliche Wirkstoffreservoirmembran können ebenfalls als retardierende Schicht eingesetzt werden.

Es können mehrere von dem Pflaster und voneinander separierte, teilbare, retardierende Wirkstoffabgaberegulierungsmittel mit einem unterschiedlichen Ausmaß an Retardierung zur Verfügung gestellt werden, vorzugsweise in demselben Kit, so dass das für die erwünschte Abgaberate entsprechende Wirkstoffabgaberegulierungsmittel aus mehreren Wirkstoffabgaberegulierungsmitteln mit unterschiedlichen Retard-Eigenschaften auszuwählen ist.

Sofern das Wirkstoffabgaberegulierungsmittel, das retardierend wirkt, bereits mit dem Pflaster lösbar verbunden ist, ist die lösbare Schutzschicht (1) zumindest in dem Umfang, in dem der wirkstoffhaltige Bereich des Pflasters bedeckt ist, wirkstoffundurchlässig, damit während der Lagerung des erfindungsgemäßen, transdermalen Wir5kstoffabgabesystems der Wirkstoff in dem wirkstoffhaltigen Bereich des Pflasters verbleibt. Erst unmittelbar vor der Applikation des Pflasters nach Entfernung der lösbaren Schutzschicht (1) wird der Kontakt direkt oder indirekt zwischen dem wirkstoffhaltigen Bereich des Pflasters und dem retardierenden Mittel ermöglicht.

Das wirkstoffhaltige Pflaster kann nach dem Reservoir- oder Matrix- System aufgebaut sein (Bauer K. H., Frömming K.-H., Führer C., Pharmazeutische Technologie, Seiten 381-383; Müller R. H., Hildebrand G. E., Pharmazeutische Technologie: Moderne Arzneiformen, Kapitel 8).

Gemäß dem Matrix-System kann das wirkstoffhaltige Pflaster vorzugsweise eine Trägerschicht, eine wirkstoffhaltige Schicht und eine Klebeschicht aufweisen, wobei die wirkstoffhaltige Schicht gleichzeitig die Klebeschicht sein kann, in der der Wirkstoff gelöst und/oder dispergiert in einer Matrix zusammen mit dem Klebstoff vorliegt. Wenn das wirkstoffhaltige Pflaster und das Wirkstoffabgaberegulierungsmittel vor der Applikation separiert vorliegen, weist das Pflaster zusätzlich noch eine lösbare Schutzschicht auf.

Als Klebstoffe für die Klebeschichten des Pflasters und des Wirkstoffabgaberegulierungsmittels werden vorzugsweise druckempfindliche Klebemittel ("pressuresensitive adhesives") eingesetzt. Beispielsweise eignen sich dafür Polymere wie Polyacrylate, Polyvinylether, Polyisobutylene (PIB), Styrol/Isopren- oder Butadien-/Styrol Copolymere oder Polyisopren Kautschuke. Weiterhin eignen sich Silikon-Klebstoffe, wie z. B. gegebenenfalls vernetzte Polydimethylsiloxane. Ferner sind Kunststoffe auf Basis von Ester von Glycinen, Glycerin oder Pentaerythrol, oder von Kohlenwasserstoffen, wie Polyterpene geeignet. Klebstoffe auf Polyacrylatbasis werden durch Polymerisation von Acrylaten, Methacrylaten, Alkylacrylaten und/oder Alkylmethacrylaten, mit gegebenenfalls weiteren α, β- ungesättigten Monomeren, wie Acrylamid, Dimethylacrylamid, Dimethylaminoethylacrylat, Hydroxyethylacrylat, Hydroxypropylacrylat, Methoxyethylacrylat, Methoxyethylmethacrylat, Acrylnitril und/oder Vinylacetat, hergestellt.

Die Klebeschichten des Pflasters und des Wirkstoffabgaberegulierungsmittels können auch Hautdurchdringungsverstärker, Füllstoffe (wie Zinkoxid oder Silika), Vernetzer, Antioxidationsmittel und/oder Lösungsmittel enthalten. Die Dicke der Klebeschichten beträgt vorzugsweise jeweils 3 bis 100 µm.

Die Trägerschicht bzw. Deckschicht des Pflasters ist vorzugsweise für die in der wirkstoffhaltigen Schicht und in der Klebeschicht enthaltenen Stoffen, insbesonders für den transdermal bzw. topisch abzugebenden Wirkstoff undurchlässig und inert, und kann auf Polymeren, wie Polyester, z. B. Polyethylenterephthalat, Polyolefinen, wie Polyethylenen, Polypropylenen oder Polybutylenen, Polycarbonaten, Polyethylenoxiden, Polyurethanen, Polystyrolen, Polyamiden, Polyimiden, Polyvinylacetaten, Polyvinylchloriden, Polyvinylidenchloriden und/oder Copolymeren wie Acrylonitril/Butadien/Styrol Copolymeren gegebenenfalls enthaltend Papierfasern, Textilfasern und/oder deren Mischungen basieren, die bei Bedarf metallisiert oder pigmentiert sein können. Die Trägerschicht bzw. Deckschicht des Pflasters kann auch aus einer Kombination aus Metallfolie und Polymerschicht bestehen. Die Dicke der Trägerschicht beträgt jeweils 3 bis 100 µm.

Die wirkstoffhaltige Matrix-Schicht des Pflasters kann matrixbildende Polymere, Hautdurchdringungsverstärker, Lösungsvermittler, Vernetzer, Stabillsatoren, Emulgatoren, Konservierungsmittel, Verdickungsmittel und/oder weitere übliche Hilfsmittel enthalten.

Als matrixbildendes Polymeres wird vorzugsweise wenigstens ein filmbildendes Polymeres ausgewählt aus der Gruppe umfassend Hydroxypropylcellulose, Carboxymethylcellulose, Polyethylene, chlorierte Polyethylene, Polypropylene, Polyurethane, Polycarbonate, Polyacrylsäureester, Polyacrylate, Polymethacrylate, Polyvinylalkohole, PolyvinylChloride, Polyvinylidenchloride, Polyvinylpyrrolidone, Polyethylenterephthalate, Polytetrafluoroethylene, Ethylen/Propylen Copolymere, Ethylen/Ethylacrylat Copolymere, Ethylen/Vinylacetat Copolymere, Ethylen/Vinylalkohol Copolymere, Ethylen/Vinyloxyethanol Copolymere, Vinylchlorid/Vinylacetat Copoly-mere, Vinylpyrrolidon/Ethylen/Vinylacetat Copolymere, Kautschuke, gummiartige, synthetische Homo-, Co- oder Blockpolymere, Silikone, Silikon-Derivate, vorzugsweise Siloxan/Methacrylat Copolymere, Cellulose-Derivate, vorzugsweise Ethylcellulose oder Celluloseether und deren Mischungen eingesetzt. Wenn die wirkstoffhaltige Schicht gleichzeitig die Klebeschicht ist, enthält sie vorzugsweise neben wenigstens einem der aufgezählten Polymeren zumindest einen der vorstehend aufgeführten Klebstoffe.

Als Lösungsvermittler können N-methyl-2-pyrrolidon, Laurylpyrrolidon, Triethanolamin, Triacetin, Diethylenglykol-monoethylether, Derivate von Fettsäuren oder Fettalkoholen, niedermolekulare, mehrwertige Alkohole wie beispielsweise Propylenglycol oder Glycerin und/oder tensidische Verbindungen verwendet werden.

Wenn das wirkstoffhaltige Pflaster nach dem Reservoir-System aufgebaut ist, kann die Reservoir-Membran aus inerten Polymeren wie z. B. Polyethylenen, Polypropylenen, Polyvinylacetaten, Polyamiden, Ethylen/Vinylacetat Copolymeren und/oder Silikonen bestehen. Mit Hilfe der Reservoir-Membran kann aus dem Reservoir bereits eine kontrollierte Freisetzung des Wirkstoffes erzielt werden.

Die wirkstoffhaltige Matrix bzw. das wirkstoffhaltige Reservoir des Pflasters kann auch Lösungsmittel, wie z. B. Wasser, Ethanol, 1-Propanol, Isopropanol, einen niedermolekularen, mehrwertigen Alkohol, beispielsweise Propylenglycol oder Glycerin, oder einen Ester, wie Isopropylmyristat, tensidische Verbindungen oder deren Mischungen enthalten.

Als Stabilisatoren für die wirkstoffhaltige Matrix bzw. das wirkstoffhaltige Reservoir können Antioxidantien, wie Vitamin E, Butylhydroxytoluol, Butylhydroxyanisol, Ascorbinsäure, Ascorbylpalmitat, und/oder Chelatbildner, wie z. B. Dinatriumethylendiamintetraessigsäure, Kalium- oder Natriumcitrat verwendet werden.

Die wirkstoffhaltige Matrix bzw. das wirkstoffhaltige Reservoir kann auch übliche Hautdurchdringungsverstärker enthalten.

Das Pflaster kann auch in einer oder mehreren Schichten wenigstens einen Weichmacher oder Hautdurchdringungsverstärker ausgewählt aus der Gruppe umfassend langkettige Alkohole, wie Dodecanol, Undecanol, Octanol, Ester von Carbonsäuren mit polyethoxylierten Alkoholen, Diester von aliphatischen Dicarbonsäuren, wie Adipinsäure, und mittelkettige Triglyceride von Caprylsäure und/oder Caprinsäure, Kokosfett, mehrwertige Alkohole, wie 1,2-Propandiol, Ester von mehrwertigen Alkoholen, wie Glycerin mit Lävulinsäure oder Caprylsäure, und veretherte mehrwertige Alkohole enthalten.

Die lösbare Schutzschicht bzw. die lösbaren Schutzschichten des erfindungsgemäßen Wirkstoffabgaberegulierungsmittels und die gegebenenfalls vorhandene lösbare Schutzschicht des Pflasters können aus Polyethylen, Polyester, Polyethylenterephthalat, Polypropylen, Polysiloxan, Polyvinylchlorid oder Polyurethan und gegebenenfalls aus behandelten Papierfasern, wie z. B. Zellophan, bestehen und gegebenenfalls wenigstens eine Silikon-, Fluorsilikon- oder Fluor-Kohlenstoffbeschichtung aufweisen. Bei einem mit dem Pflaster lösbar verbundenen Wirkstoffabgaberegulierungsmittel sind vorzugsweise beide Oberflächen der Schutzschicht so ausgerüstet.

Die Herstellung des erfindungsgemäßen Wirkstoffabgaberegulierungsmittels bzw. des Pflaster kann nach bekannten Herstellungsverfahren erfolgen, die die Verfahrensschritte Laminieren, Coextrudieren, Stanzen, Delaminieren, Abwickeln, Schneiden, Wiederaufwickeln, Montieren und/oder Dosieren (Verpackungs-Rundschau 4/2002, 83-84) umfassen.

### Beispiele

### Beispiel 1

### a) Herstellung eines Buprenorphin-.haltigen Pflasters

1139 g einer 48 Gew.%igen Polyacrylatlösung eines selbstvernetzenden Acrylatcopolymeren aus 2-Ethylhexylacrylat, Vinylacetat, Acrylsäure (Lösemittel: Ethylacetat:Heptan:Isopropanol:Toluol:Acetylacetonat im Verhältnis von 37:26:26:4:1), 100 g Lävulinsäure, 150 g Oleylacetat, 100 g Polyvinylpyrrolidon, 150 g Ethanol, 200 g Ethylacetat und 100 g Buprenorphinbase wurden homogenisiert. Man rührt etwa zwei Stunden und kontrollierte visuell, ob alle Feststoffe gelöst waren. Man kontrollierte außerdem den Verdunstungsverlust durch Zurückwiegen und ergänzte gegebenenfalls den Lösemittelverlust durch Zugabe von Ethylacetat.

Als Deckschicht wurde eine 420 mm breite, transparente Polyesterfolie mit der vorstehend beschriebenen Mischung so beschichtet, dass das Flächengewicht der getrockneten Klebeschicht bei 80 g/m² lag.

Man entfernt die Lösemittel durch Trocknen mit erwärmter Luft, die über die feuchte Bahn geleitet wurde. Durch die Wärmebehandlung verdampften die Lösemittel. Abschließend deckte man die wirkstoffhaltige Klebeschicht mit einer 15 µm dicken Polyesterfolie ab, die durch eine Silikonbehandlung wieder ablösbar war. Mit geeigneten Schneidewerkzeugen stanzte man eine für die vorgesehene Wirkstoffmenge entsprechende Fläche aus.

### b) Herstellung eines Wirkstoffabgaberegulierungsmittels mit dem folgenden Schichtaufbau:

- Eine lösbare Schutzschicht
- Eine Klebeschicht
- Eine Wirkstoffsperrschicht
- Eine Klebeschicht
- Eine lösbare Schutzschicht

Zur Herstellung des erfindungsgemäßen Wirkstoffabgaberegulierungsmittels wurde eine Polyethylenterephthalat-Folie mit einer Dicke von 75 µm als Wirkstoffsperrschicht in einem Ericsson-Filmziehgerät (der Fa. Ericsson GmbH & Co. KG, Hemer, Deutschland) eingespannt und mit der unter Beispiel 1 a) mit Ausnahme von Buprenorphin beschriebenen Mischung beschichtet und 2 Stunden getrocknet, wodurch man eine Klebeschicht mit einer Dicke von 90 µm erhielt. Diese Klebeschicht wurde mit einer einseitig silikonisierten Folie auf Polyethylenterephthalatbasis mit der silikonisierten Seite lösbar verbunden.

Auf der noch freien zweiten Oberfläche der Sperrschicht wurde der Vorgang wiederholt, so dass die Wirkstoffsperrschicht beidseitig eine Klebeschicht mit einer Dicke von 90 µm und einer damit lösbar verbundenen Schutzschicht aufwies.

Mit Hilfe eines geeigneten Stanzwerkzeuges wurde nicht nur das Wirkstoffabgaberegulierungsmittel mit einer Größe gemäß der Größe der nach Beispiel 1 a) hergestellten Pflaster ausgestanzt, sondern auch vier gleich große Quadrate auf dem Wirkstoffabgaberegulierungsmittel durch Perforationslinien erzeugt.

Durch Ablösen eines Viertels des Wirkstoffabgaberegulierungsmittels entlang der vorgesehenen Perforationslinien und Verbinden einer Klebeschicht nach Ablösen der lösbaren Schutzschicht des verbleibenden Wirkstoffabgaberegutierungsmittels mit dem wirkstoffhaltigen Bereich des nach Beispiel 1 a) hergestellten Pflasters war es möglich, die Dosierung und damit die Abgabe des Wirkstoffes um ¼ zu verringern.

### Referenz Beispiel 2

a) Herstellung eines Buprenorphin-Pflasters
   Die Herstellung des Pflasters erfolgte wie in Beispiel 1a) angegeben.
b) Herstellung eines Wirkstoffabgaberegulierungsmittels mit folgendem Schichtaufbau:
   - eine lösbare Schutzschicht
   - eine Klebeschicht
   - eine lösbare Schutzschicht

Zur Herstellung des erfindungsgemäßen Wirkstoffabgaberegulierungsmittels wurde eine einseitig siikonisierte Polyethylenterephthalat-Folie mit einer Dicke von 36 µm als lösbare Schutzschicht in einem Ericsson-Filmziehgerät (der Fa. Ericsson GmbH & Co. KG, Herma, Deutschland) eingespannt und mit der unter Beispiel 1 a) mit Ausnahme von Buprenorphin beschriebenen Mischung beschichtet und 2 Stunden getrocknet, wodurch man eine Klebeschicht mit einer Dicke von 15 µm erhielt. Diese Klebeschicht wurde mit einer einseitig silikonisierten Folie auf Polyethylenterephthalatbasis lösbar verbunden.

Mit Hilfe eines geeigneten Stanzwerkzeuges wurde das Wirkstoffabgaberegulierungsmittel mit einer Größe entsprechend der nach Beispiel 2a) hergestellten Pflaster gestanzt. Nach dem Entfernen der lösbaren Schutzschicht von der Klebeschicht des nach Beispiel 2a) hergestellten Pflasters und einer lösbaren Schutzschicht von der Klebeschicht des nach Beispiel 2b) hergestellten Wirkstoffabgaberegulierungsmittels wurden die so freigelegten Klebeschichten miteinander verbunden. Dadurch war der wirkstoffhaltige Bereich des Pflasters zu 100% mit dem Regulierungsmittel bedeckt.

Die Prüfung der Wirksamkeit des Wirkstoffabgaberegulierungsmittels erfolgte mit Hilfe des "tail flick tests", dem pharmakologischen Schmerzprüftest an Ratten. Es wurde dieser Test sowohl mit Pflastern gemäß 2a) ohne Mitverwendung eines Wirkstoffabgaberegulierungsmittels und mit Pflastern gemäß 2a), unter Mitverwendung eines Wirkstoffabgaberegulierungsmittels gemäß 2b) durchgeführt. Die entsprechenden Testergebnisse sind in der nachfolgenden Tabelle zusammengestellt.

| | Buprenorphin Pflaster ohne Regulierungsmittel | Buprenorphin Pflaster mit Wirkstoffabgaberegulierungsmittel |
|---|---|---|
| Nach 2 Stunden | Keine Messung | 33% MPE |
| Nach 4 Stunden | 95% MPE | 61% MPE |
| Nach 6 Stunden | 96% MPE | 81% MPE |
| Nach 8 Stunden | 100% MPE | 100% MPE |
| Nach 12 Stunden | 98% MPE | 100% MPE |

Angaben in MPE (=maximum possible effect), wobei eine maximale analgetische Wirkung 100% MPE entspricht.

Die Ergebnisse zeigen deutlich, dass die Wirkstoffabgabe in den ersten 6 Stunden nach der Applikation verzögert erfolgt.

## Patentansprüche

1. Wirkstoffabgabesystem zur systemischen oder topischen Abgabe eines Wirkstoffes durch und/oder an die Haut eines menschlichen oder tierischen Organismus umfassend
a) ein wirkstoffhaltiges Pflaster und
b) wenigstens ein teilbares, wirkstoffundurchlässiges oder wenigstens ein die Wirkstoffabgabe retardierendes, teilbares Wirkstoffabgaberegulierungsmittel, das von dem Pflaster separiert vorliegt, oder ein die Wirkstoffabgabe retardierendes Wirkstoffabgaberegulierungsmittel, das bereits mit dem Pflaster lösbar verbunden ist.

2. Wirkstoffabgabesystem nach Anspruch 1, **dadurch gekennzeichnet, dass** das separiert vorliegende, teilbare wirkstoffundurchlässige Wirkstoffabgaberegulierungsmittel mehrschichtig ist und folgenden Schichtaufbau umfasst:
- eine lösbare Schutzschicht
- eine Klebeschicht
- eine Wirkstoffsperrschicht.

3. Wirkstoffabgabesystem nach Anspruch 2, **dadurch gekennzeichnet, dass** das teilbare, separiert vom Pflaster vorliegende, wirkstoffundurchlässige Wirkstoffabgaberegulierungsmittel folgenden Schichtaufbau umfasst:
- eine lösbare Schutzschicht
- eine Klebeschicht
- eine Wirkstoffsperrschicht
- eine weitere Klebeschicht zum Verbinden mit der Haut
- eine weitere lösbare Schutzschicht.

4. Wirkstoffabgabesystem nach Anspruch 1, **dadurch gekennzeichnet, dass** das die Wirkstoffabgabe retardierendes Wirkstoffabgaberegulierungsmittel, das bereits mit dem Pflaster lösbar verbunden ist, teilbar ist.

5. Wirkstoffabgabesystem nach Anspruch 1 oder 4, **dadurch gekennzeichnet, dass** das die Wirkstoffabgabe retardierende Wirkstoffabgaberegulierungsmittel mehrschichtig ist und folgenden Schichtaufbau umfasst:
- eine lösbare Schutzschicht
- eine die Wirkstoffabgabe retardierende Klebeschicht
- eine weitere Klebeschicht und
- eine weitere lösbare Schutzschicht.

6. Wirkstoffabgabesystem nach Anspruch 5, **dadurch gekennzeichnet, dass** das separiert vom Pflaster vorliegende, retardierende Wirkstoffabgaberegulierungsmittel folgenden Schichtaufbau aufweist:
- eine lösbare Schutzschicht
- eine Klebeschicht
- eine die Wirkstoffabgabe retardierende Schicht
- eine Klebeschicht
- eine lösbare Schutzschicht.

7. Wirkstoffabgabesystem nach Anspruch 5, **dadurch gekennzeichnet, dass** das separiert vom Pflaster vorliegende, retardierende Wirkstoffabgaberegulierungsmittel folgenden Schichtaufbau aufweist:
- eine lösbare Schutzschicht
- eine Klebeschicht, die als eine die Wirkstoffabgabe retardierende Schicht funktioniert,
- eine lösbare Schutzschicht.

8. Wirkstoffabgabesystem nach Anspruch 5, **dadurch gekennzeichnet, dass** das die Wirkstoffabgabe retardierende Wirkstoffabgaberegulierungsmittel mit dem Pflaster bereits lösbar verbunden folgenden Schichtaufbau aufweist:
- eine lösbare Schutzschicht
- eine Klebeschicht
- eine die Wirkstoffabgabe retardierende Schicht
- eine Klebeschicht zum Verbinden mit der Haut
- eine lösbare Schutzschicht .

9. Wirkstoffabgabesystem nach Anspruch 5, **dadurch gekennzeichnet, dass** das die Wirkstoffabgabe retardierende Wirkstoffabgaberegulierungsmittel, das mit dem Pflaster bereits lösbar verbunden ist, folgenden Schichtaufbau aufweist:
- eine lösbare Schutzschicht
- eine Klebeschicht, die als eine die Wirkstoffabgabe retardierende Schicht funktioniert,
- eine lösbare Schutzschicht.

10. Wirkstoffabgabesystem nach einem der Ansprüche 1 bis 9, **dadurch gekennzeichnet, dass** das teilbare Wirkstoffabgaberegulierungsmittel über vorgegebene Trennungsmöglichkeiten teilbar ist.

11. Wirkstoffabgabesystem nach Anspruch 10, **dadurch gekennzeichnet, dass** das teilbare Wirkstoffabgaberegulierungsmittel über Schwächungslinien, Perforationslinien oder Schneidemarkierungen teilbar ist.

12. Wirkstoffabgabesystem nach einem der Ansprüche 1 bis 3, 10, oder 11, **dadurch gekennzeichnet, dass** das wirkstoffundurchlässige Wirkstoffabgaberegulierungsmittel so teilbar ist, dass jeweils 10 bis 90% der Oberfläche des wirkstoffhaltigen Bereichs des Pflasters bedeckbar sind.

13. Wirkstoffabgabesystem nach einem der Ansprüche 1 bis 7, **dadurch gekennzeichnet, dass** es wenigstens eines vom Pflaster separiertes Wirkstoffabgaberegulierungsmittel umfasst.

14. Wirkstoffabgabesystem nach Anspruch 13, **dadurch gekennzeichnet, dass** es mehr als ein separiertes Wirkstoffabgaberegulierungsmittel in unterschiedlicher Größe umfasst.

15. Wirkstoffabgabesystem nach einem der Ansprüche 10 bis 14, **dadurch gekennzeichnet, dass** die voneinander trennbaren Teile eines teilbaren Wirkstoffabgaberegulierungsmittels oder die voneinander separat verpackten Wirkstoffabgaberegulierungsmittel eine unterschiedliche Markierung zur Unterscheidung aufweisen.

16. Wirkstoffabgabesystem nach Anspruch 15, **dadurch gekennzeichnet, dass** die voneinander trennbaren Teile eines teilbaren Wirkstoffabgaberegulierungsmittels oder die voneinander separat verpackten Wirkstoffabgaberegulierungsmittel eine unterschiedliche Färbung zur Unterscheidung aufweisen.

17. Wirkstoffabgabesystem nach einem der Ansprüche 1, 4, 5, 8 bis 12, 15 oder 16, **dadurch gekennzeichnet, dass** das Wirkstoffabgaberegulierungsmittel vor der Applikation zunächst ablösbar den gesamten wirkstoffhaltigen Bereich des Pflasters bedeckt.

18. Wirkstoffabgabesystem nach einem der Ansprüche 1, 4 bis 7, 10, 11, 13 bis 15 oder 16, **dadurch gekennzeichnet, dass** mit Hilfe eines separiert verpackten, teilbaren, die Wirkstoffabgabe retardierenden Wirkstoffabgaberegulierungsmittels 10 bis 100% des wirkstoffhaltigen Bereichs des Pflasters abgedeckt werden können.

19. Wirkstoffabgabesystem nach einem der Ansprüche 1 bis 18, **dadurch gekennzeichnet, dass** das Pflaster ein wirkstoffhaltiges Reservoir oder eine wirkstoffhaltige Matrix aufweist.

20. Wirkstoffabgabesystem nach einem der Ansprüche 1 bis 19, **dadurch gekennzeichnet, dass** es als Kit vorliegt.

## Claims

1. Active ingredient release system for the systemic or topical release of an active ingredient through and/or onto the skin of a human or animal organism, comprising
a) a plaster containing the active ingredient, and
b) at least one divisible active ingredient release regulator impermeable to the active ingredient, or at least one divisible active ingredient release regulator that retards the release of the active ingredient and is present separate from the plaster, or an active ingredient release regulator that retards the release of the active ingredient and is already detachably bonded to the plaster.

2. Active ingredient release system according to Claim 1, **characterised in that** the separate, divisible active ingredient release regulator impermeable to the active ingredient is multilayered and has the following layer structure:
- a detachable protective layer
- an adhesive layer
- an active ingredient barrier layer.

3. Active ingredient release system according to Claim 2, **characterised in that** the divisible active ingredient release regulator that is impermeable to the active ingredient and separate from the plaster has the following layer structure:
- a detachable protective layer
- an adhesive layer
- an active ingredient barrier layer
- a further adhesive layer for bonding to the skin
- a further detachable protective layer.

4. Active ingredient release system according to Claim 1, **characterised in that** the active ingredient release regulator that retards the release of the active ingredient and is already detachably bonded to the plaster is divisible.

5. Active ingredient release system according to Claim 1 or 4, **characterised in that** the active ingredient release regulator that retards the release of the active ingredient is multilayered and has the following layer structure:
- a detachable protective layer
- an adhesive layer that retards the release of the active ingredient
- a further adhesive layer, and
- a further detachable protective layer.

6. Active ingredient release system according to Claim 5, **characterised in that** the retarding active ingredient release regulator separate from the plaster has the following layer structure:
- a detachable protective layer
- an adhesive layer
- a layer that retards the release of the active ingredient
- an adhesive layer
- a detachable protective layer.

7. Active ingredient release system according to Claim 5, **characterised in that** the retarding active ingredient release regulator separate from the plaster has the following layer structure:
- a detachable protective layer
- an adhesive layer acting as a layer that retards the release of the active ingredient
- a detachable protective layer.

8. Active ingredient release system according to Claim 5, **characterised in that** the active ingredient release regulator that retards the release of the active ingredient and is already detachably bonded to the plaster has the following layer structure:
- a detachable protective layer
- an adhesive layer
- a layer that retards the release of the active ingredient
- an adhesive layer for bonding to the skin
- a detachable protective layer.

9. Active ingredient release system according to Claim 5, **characterised in that** the active ingredient release regulator that retards the release of the active ingredient and is already detachably bonded to the plaster has the following layer structure:
- a detachable protective layer
- an adhesive layer acting as a layer that retards the release of the active ingredient
- a detachable protective layer.

10. Active ingredient release system according to one of Claims 1 to 9, **characterised in that** the divisible active ingredient release regulator is divisible by predetermined separating means.

11. Active ingredient release system according to Claim 10, **characterised in that** the divisible active ingredient release regulator is divisible along lines of weakness, perforation lines or cutting marks.

12. Active ingredient release system according to one of Claims 1 to 3, 10 or 11, **characterised in that** the active ingredient release regulator impermeable to the active ingredient is divisible so that in each case 10 to 90% of the surface of the region of the plaster that contains the active ingredient can be covered.

13. Active ingredient release system according to one of Claims 1 to 7, **characterised in that** it comprises at least one active ingredient release regulator separate from the plaster.

14. Active ingredient release system according to Claim 13, **characterised in that** it comprises more than one separate active ingredient release regulator of different size.

15. Active ingredient release system according to one of Claims 10 to 14, **characterised in that** the parts of a divisible active ingredient release regulator that can be separated from one another, or the active ingredient release regulators packaged separately from one another, carry different distinguishing markings.

16. Active ingredient release system according to Claim 15, **characterised in that** the parts of a divisible active ingredient release regulator that can be separated from one another, or the active ingredient release regulators packaged separately from one another, have different distinguishing colourings.

17. Active ingredient release system according to one of Claims 1, 4, 5, 8 to 12, 15 or 16, **characterised in that** the active ingredient release regulator before application initially detachably covers the entire region of the plaster that contains the active ingredient.

18. Active ingredient release system according to one of Claims 1, 4 to 7, 10, 11, 13 to 15 or 16, **characterised in that** 10 to 100% of the region of the plaster that contains the active ingredient can be covered with a separately packaged, divisible active ingredient release regulator that retards the release of the active ingredient.

19. Active ingredient release system according to one of Claims 1 to 18, **characterised in that** the plaster has a reservoir that contains the active ingredient or a matrix that contains the active ingredient.

20. Active ingredient release system according to one of Claims 1 to 19, **characterised in that** it is in the form of a kit.

## Revendications

1. Système de libération de substances actives destiné à la libération systémique ou topique d'une substance active via et/ou sur la peau d'un organisme humain ou animal, comprenant
a) un pansement contenant des substances actives et
b) au moins un agent de régulation de la libération de substances actives, sécable, imperméable aux substances actives ou au moins un agent de régulation de la libération de substances actives, sécable, retardant la libération des substances actives, qui se trouve sous une forme séparée du pansement ou un agent de régulation de la libération de substances actives retardant la libération des substances actives, qui est déjà assemblé de manière amovible avec le pansement.

2. Système de libération de substances actives selon la revendication 1, **caractérisé en ce que** l'agent de régulation de la libération de substances actives se trouvant sous forme séparée, sécable, imperméable aux substances actives est à plusieurs couches et comprend la structure à couches suivante :
- une couche de protection amovible
- une couche adhésive
- une couche de blocage des substances actives.

3. Système de libération de substances actives selon la revendication 2, **caractérisé en ce que** l'agent de régulation de la libération de substances actives, sécable, se trouvant sous forme séparée du pansement, imperméable aux substances actives comprend la structure à couches suivante :
- une couche de protection amovible
- une couche adhésive
- une couche de blocage des substances actives
- une autre couche adhésive pour l'assemblage avec la peau
- une autre couche de protection amovible.

4. Système de libération de substances actives selon la revendication 1, **caractérisé en ce que** l'agent de régulation de la libération de substances actives retardant la libération des substances actives, qui est déjà assemblé de manière amovible avec le pansement, est sécable.

5. Système de libération de substances actives selon la revendication 1 ou 4, **caractérisé en ce que** l'agent de régulation de la libération de substances actives retardant la libération des substances actives est à plusieurs couches et comprend la structure à couches suivante :
- une couche de protection amovible
- une couche adhésive retardant la libération des substances actives
- une autre couche adhésive et
- une autre couche de protection amovible.

6. Système de libération de substances actives selon la revendication 5, **caractérisé en ce que** l'agent de régulation de la libération de substances actives, se trouvant sous forme séparée du pansement, à effet retardateur, comprend la structure à couches suivante :
- une couche de protection amovible
- une couche adhésive
- une couche retardant la libération des substances actives
- une couche adhésive
- une couche de protection amovible.

7. Système de libération de substances actives selon la revendication 5, **caractérisé en ce que** l'agent de régulation de la libération de substances actives, se trouvant sous forme séparée du pansement, à effet retardateur, comprend la structure à couches suivante :
- une couche de protection amovible
- une couche adhésive, qui agit comme couche retardant la libération des substances actives,
- une couche de protection amovible.

8. Système de libération de substances actives selon la revendication 5, **caractérisé en ce que** l'agent de régulation de la libération des substances actives, retardant la libération des substances actives déjà assemblé de manière amovible avec le pansement présente la structure à couches suivante :
- une couche de protection amovible
- une couche adhésive
- une couche retardant la libération des substances actives
- une couche adhésive pour l'assemblage avec la peau
- une couche de protection amovible.

9. Système de libération de substances actives selon la revendication 5, **caractérisé en ce que** l'agent de régulation de la libération des substances actives, retardant la libération des substances actives, qui est déjà assemblé de manière amovible avec le pansement présente la structure à couches suivante :
- une couche de protection amovible
- une couche adhésive, qui agit comme couche retardant la libération des substances actives,
- une couche de protection amovible.

10. Système de libération de substances actives selon l'une quelconque des revendications 1 à 9, **caractérisé en ce que** l'agent de régulation de substances actives, sécable, peut être divisé par des possibilités de séparation fixées à l'avance.

11. Système de libération de substances actives selon la revendication 10, **caractérisé en ce que** l'agent de régulation de la libération de substances actives, sécable, peut être divisé via des lignes d'affaiblissement, des lignes de perforations ou des marquages de découpe.

12. Système de libération de substances actives selon l'une quelconque des revendications 1 à 3, 10 ou 11, **caractérisé en ce que** le système de régulation de la libération de substances actives, imperméable aux substances actives peut être divisé de manière telle qu'à chaque fois 10 à 90% de la surface de la zone contenant les substances actives du pansement peuvent être recouverts.

13. Système de libération de substances actives selon l'une quelconque des revendications 1 à 7, **caractérisé en ce qu'**il comprend au moins un agent de régulation de la libération de substances actives séparé du pansement.

14. Système de libération de substances actives selon la revendication 13, **caractérisé en ce qu'**il comprend plus d'un agent de régulation de la libération de substances actives séparé de tailles différentes.

15. Système de libération de substances actives selon l'une quelconque des revendications 10 à 14, **caractérisé en ce que** les parties séparables les unes des autres d'un agent de régulation de la libération de substances actives sécable ou les agents de régulation de la libération de substances actives emballés séparément les uns des autres présentent un marquage différent en vue de la différenciation.

16. Système de libération de substances actives selon la revendication 15, **caractérisé en ce que** les parties séparables les unes des autres d'un agent de régulation de la libération de substances actives sécable ou les agents de régulation de la libération de substances actives emballés séparément les uns des autres présentent une coloration différente en vue de la différenciation.

17. Système de libération de substances actives selon l'une quelconque des revendications 1, 4, 5, 8 à 12, 15 ou 16, **caractérisé en ce que** l'agent de régulation de la libération de substances actives recouvre avant l'application, en premier lieu, de manière amovible, la totalité de la zone contenant les substances actives du pansement.

18. Système de libération de substances actives selon l'une quelconque des revendications 1, 4 à 7, 10, 11, 13 à 15 ou 16, **caractérisé en ce qu'**à l'aide d'un agent de régulation de la libération de substances actives emballé séparément, sécable, retardant la libération de substances actives, on peut recouvrir 10 à 100% de la zone contenant des substances actives du pansement.

19. Système de libération de substances actives selon l'une quelconque des revendications 1 à 18, **caractérisé en ce que** le pansement présente un réservoir contenant des substances actives ou une matrice contenant des substances actives.

20. Système de libération de substances actives selon l'une quelconque des revendications 1 à 19, **caractérisé en ce qu'**il se trouve sous forme de kit.
